# EUROPEAN PATENT APPLICATION

(11) **EP 1 808 690 A1**
(43) Date of publication of application: **18.07.2007**
(21) Application number: 06024553.7
(22) Date of filing: 27.11.2006
(51) Int. Cl.: G01N 21/89, G01N 33/36

(54) **Foreign matter detecting device and textile machine and foreign matter detecting method**

(30) Priority: 16.01.2006 JP 2006007510; 15.02.2006 JP 2006037693
(71) Applicant: MURATA KIKAI KABUSHIKI KAISHA, Minami-ku, Kyoto-shi, Kyoto 601 (JP)
(72) Inventor: Nakade, Kazuhiko, Fushimi-ku, Kyoto-shi, Kyoto (JP)
(74) Representative: Liedl, Christine

(57) **Abstract**

When foreign matters mixed in a yarn are to be detected and if any of the foreign matters is desirably to be left, avoiding detecting the foreign matter to be desirably left reduces detection accuracy. To solve this problem, the present invention provides a foreign matter detecting device (32) which alternately irradiates a spun yarn (9) formed of cotton fibers with light (direct light Da, Db) from LEDs (37a,37b) and which measures intensity of reflected light (Rb,Ra) from the spun yarn (9) to detect presence or absence of a foreign matter mixed in the spun yarn (9) on the basis of the intensity information, wherein the light source color of the LEDs (37a,37b) is yellow or yellowish orange, that is, in a hue ring, the light source color of the LEDs (37a,37b) is the same as or similar to the color of trash of stems or leaves of cotton fibers, a foreign matter to be desirably left instead being removed.

## Description

### Field of the Invention

The present invention relates to a foreign matter detecting device that detects the presence or absence of a foreign matter mixed in a yarn, and a textile machine using the foreign matter detecting device, and a foreign matter detecting method.

### Background of the Invention

In the manufacture of yarns such as spun yarns or fiber bundles, a foreign matter may be mixed in fibers, which are a raw material for the yarns, and may not be removed during the intermediate stage of a manufacturing process. The foreign matter may remain even after a yarn has been formed using the fibers as a raw material. Since the remaining foreign matter degrades the yarn, accordingly, it is necessary to detect the presence or absence of a foreign matter mixed in the yarn and to cut and remove the part of the yarn in which a foreign matter is mixed.

For cotton yarns (yarns formed of cotton fibers), fibers (raw cotton obtained from a cotton tree) as a material contains trash of cotton stems or leaves as a foreign matter. The trash is removed from the raw cotton during a carding process or the like but may partly remain. Discolored fibers are also a foreign matter.

As disclosed in the Unexamined Japanese Patent Application Publication (Tokkai-Sho) No. 61-292046, a sensor has been known which detects foreign matter mixed in a yarn.

This sensor irradiates the yarn with light from a light source and measures the intensity of reflected light to determine the presence or absence of a foreign matter mixed in the yarn on the basis of the intensity information.

The sensor utilizes the difference of color of the outer surface of the light irradiation target, that is, the difference in light reflectivity between fibers as a raw material and a foreign matter. The difference in light reflectivity between the fibers and the foreign matter (including discolored fibers) makes the intensity of reflected light from the fibers different from that from the foreign matter.

As a light source, green light (Unexamined Japanese Patent Application Publication (Tokkai-Sho) No. 61-292046: this light has a peak at red), yellowish green light, or the like is utilized for economical reasons.

This sensor is configured so that the intensity of reflected light does not vary depending on the thickness of the yarn. Specifically, a background (reflecting portion) having the same reflectivity as that of fibers as a raw material is provided behind the yarn. Then, the intensity of the entire reflected light from both yarn and reflecting portion is measured. This results in reflected light of the same intensity regardless of the yarn thickness where the yarn contains no foreign matter.

For cotton yarns, a dying process or the like during a postprocess dyes remaining trash of stems or leaves together with the yarn, making the trash unnoticeable. Accordingly, the trash need not necessarily be removed. On the contrary, an attempt to completely remove the trash reduces the speed (spun yarn spinning speed or fasciated yarn spinning speed) at which cotton yarns are manufactured from raw cotton, degrading production efficiency or increasing the amount of waste material.

Thus, the yarn may contain a foreign matter to be desirably left instead of being removed even if the foreign matter is different from the fibers as a raw material.

However, conventional sensors such as the one disclosed in the Unexamined Japanese Patent Application Publication (Tokkai-Sho) No. 61-292046 recognize all the foreign matters having colors different from that of the fibers as a raw material, as foreign matters. Of course, since the trash and the discolored fibers are different in color and in the intensity of reflected light, it is possible to avoid recognizing the trash as a foreign matter by appropriately setting a threshold beyond which the presence of a foreign matter is recognized. However, setting such a threshold may be complicated or may reduce detection accuracy.

A problem to be solved by the present invention is that when foreign matters mixed in a yarn are to be detected and where any of the foreign matters is desirably to be left, avoiding detecting the foreign matter to be desirably left reduces detection accuracy.

### Summary of the Invention

A description has been given of the problem to be solved by the present invention. Now, a description will be given of means for solving the problem.

According to Claim 1, there is provided a foreign matter detecting device which irradiates a yarn formed of white fibers with light from a light source and which measures intensity of reflected light from the yarn to detect presence or absence of a foreign matter mixed in the yarn on the basis of the intensity information,
wherein in a hue ring, a light source color of the light source is the same as or similar to a color of a foreign matter to be desirably left instead being removed.

The yarn may be a spun yarn or a fasciated spun yarn or a simple fiber bundle.

The white fibers are cotton fibers, wool, or the like, and the white fibers used are not limited as long as yarns can be made from these fibers.

To change the light source color, it is possible to change the coloring of the light source itself or to provide a color filter in the light source to change the color of radiated light.

This configuration has the following function.

The reflectivity of the fibers forming the yarn is almost the same as that of the foreign matter to be desirably left instead of being removed. The intensity of reflected light from the fibers forming the yarn is thus almost the same as that from the foreign matter to be desirably left instead of being removed. This makes the intensity information unlikely to contain information useful for distinguishing the fibers forming the yarn from the foreign matter to be desirably left instead of being removed.

A foreign matter detecting device according to Claim 2 is the foreign matter detecting device set forth in Claim 1, wherein the yarn is a spun yarn formed of cotton fibers,
the foreign matter to be desirably left instead of being removed is trash of cotton leaves or stems which is yellow or yellowish orange, and
the light source color is yellow or yellowish orange.

This configuration has the following function.

The foreign matter detecting device that detects the presence or absence of a foreign matter on the basis of intensity information is unlikely to distinguish the fibers forming the yarn from the foreign matter to be desirably left instead of being removed.

According to Claim 3, there is provided a textile machine comprising:
a foreign matter detecting device according to Claim 1 or Claim 2;
a cutting device for the yarn which operates on the basis of detection of the foreign matter by the foreign matter detecting device; and
a winding device that winds the yarn.

This configuration has the following function.

Yarn winding is carried out without allowing the cutting device to remove the foreign matter to be desirably left.

According to Claim 4, there is provided a foreign matter detecting method of irradiating a yarn formed of white fibers with light from a light source and measuring intensity of reflected light from the yarn to detect presence or absence of a foreign matter mixed in the yarn on the basis of the intensity information,
wherein in a hue ring, a light source color of the light source is the same as or similar to a color of foreign matter to be desirably left instead being removed.

This configuration has the following function.

The reflectivity of the fibers forming the yarn is almost the same as that of the foreign matter to be desirably left instead of being removed. The intensity of reflected light from the fibers forming the yarn is thus almost the same as that from the foreign matter to be desirably left instead of being removed. This makes the intensity information unlikely to contain information useful for distinguishing the fibers forming the yarn from the foreign matter to be desirably left instead of being removed.

The present invention exerts the following effects.

According to Claim 1, the intensity information is unlikely to contain information useful for distinguishing the fibers forming the yarn from the foreign matter to be desirably left instead of being removed. Consequently, the foreign matter to be desirably left instead of being removed is unlikely to be detected even with a strict threshold set to detect a foreign matter to be removed. That is, avoiding detection of the foreign matter to be desirably left does not reduce the accuracy with which the foreign matter to be removed is detected.

According to Claim 2, avoiding detection of trash does not reduce the accuracy with which the foreign matter to be removed such as discolored fibers is detected.

Claim 3 produces no only the effect of Claim 1 or Claim 2 but also the following effect.

The speed (spun yarn spinning speed, fasciated yarn spinning speed, drawing speed, or the like) at which yarns are manufactured from a raw material is prevented from decreasing. This in turn prevents a decrease in production efficiency and an increase in the amount of waste material.

According to Claim 4, the intensity information is unlikely to contain information useful for distinguishing the fibers forming the yarn from the foreign matter to be desirably left instead of being removed. Consequently, the foreign matter to be desirably left instead of being removed is unlikely to be detected even with a strict threshold set to detect foreign matter to be removed. That is, avoiding detection of the foreign matter to be desirably left does not reduce the accuracy with which the foreign matter to be removed is detected.

Other features, elements, processes, steps, characteristics and advantages of the present invention will become more apparent from the following detailed description of preferred embodiments of the present invention with reference to the attached drawings.

### Brief Description of the Drawings

Figure 1 is a configuration of a pneumatic spinning machine.
Figure 2A is a horizontal sectional view of a yarn defect detecting device and Figure 2B is a chart of a yarn defect detecting device.
Figure 3 is a diagram showing the spectrum of a yellow light source.
Figure 4 is a diagram showing an example of how to set a threshold when the yellow light source is used.
Figure 5 is a diagram showing an example of how to set a threshold when a green light source is used.
Figure 6 is a diagram showing a comparison of the number of cuts between the case with the yellow light source and the case with the green light source.

### Detailed Description of the Preferred Embodiment

An embodiment of present invention will be described with reference to the drawings.

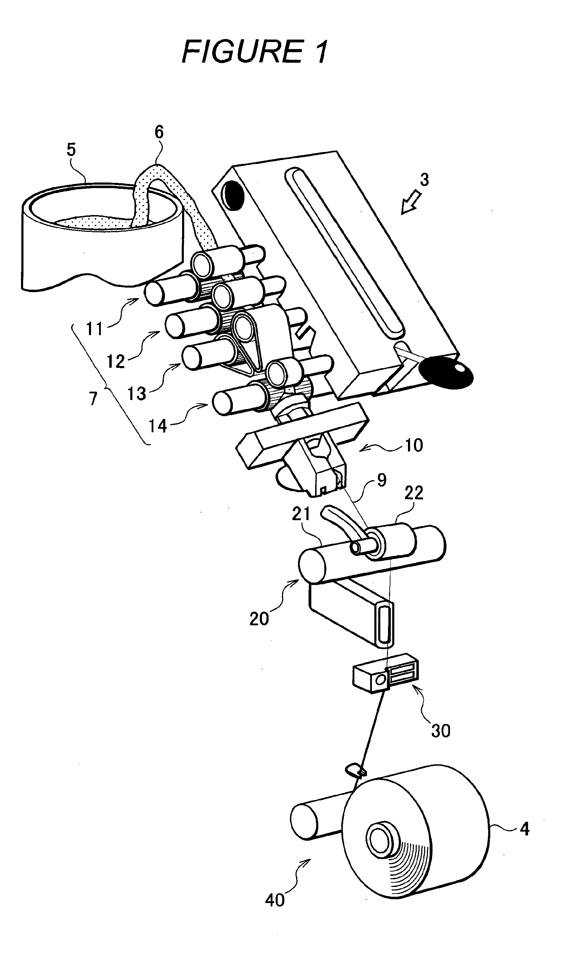

First, a pneumatic spinning machine 3 that spins cotton yarns will be described with reference to Figure 1.

The pneumatic spinning machine 3 is a device that manufactures a spun yarn 9 of cotton fibers from a sliver 6 of cotton fibers.

The pneumatic spinning machine 3 has a sliver can 5, a draft device 7, a pneumatic spinning device 10, a yarn feeding device 20, a yarn defect detecting device 30, and a winding device 40 sequentially arranged along a path (hereinafter referred to as a manufacturing path) along which the spun yarn 9 is manufactured from the sliver 6; a sliver can 5 is located at the most upstream position of the manufacturing path.

The sliver can (sliver accommodating vessel) 5 accommodates slivers generated by a drawing frame.

The draft device 7 comprises four pairs of draft rollers that sandwiches and draws the sliver 6 between them. The four pairs of draft rollers are arranged in the direction in which the sliver 6 is conveyed and include a back roller pair 11, a third roller pair 12, a second roller pair 13, and a front roller pair 14.

The pneumatic spinning device 10 allows whirling currents to act on the fiber bundle of the sliver 6 in an air spinning nozzle (which is built into the pneumatic spinning device 10) to manufacture the spun yarn (or fasciated spun yarn) 9.

The pneumatic spinning machine 3 according to the present embodiment has a spinning speed of 300 to 400 m/min, which is about 20 times as high as that of a ring spinning machine (20 to 30 m/min). The pneumatic spinning machine 3 is thus able to achieve high-speed spinning.

The yarn feeding device 20 feeds the spun yarn 9 manufactured by the pneumatic spinning device 10 out to the winding device 40, and the yarn feeding device 20 comprises a delivery roller 21 and a nip roller 22 which nips and feeds out the spun yarn 9.

The yarn defect detecting device 30 detects yarn defects in the spun yarn 9 being fed to the winding device 40. On the basis of the yarn defect detection information from the yarn defect detecting device 30, yarn defect parts are removed to prevent an improper yarn from being wound into a package 4. In this case, the yarn defect detecting device 30 comprises a cutting device (not shown in the drawings) that cuts the spun yarn 9 in response to detection of a yarn defect.

The pneumatic spinning machine 3 also comprises a yarn splicing device (not shown in the drawings) that splices the opposite ends of the spun yarn 9 that has been cut to remove a yarn defect part.

The winding device 40 traverses the spun yarn 9 manufactured by the pneumatic spinning device 10 in the axial direction of a bobbin to wind it to form a package 4.

The yarn defect detecting device 30 will be described in further detail.

The yarn defect detecting device 30 is configured to be able to detect two types of yarn defects, that is, parts with abnormal yarn thicknesses such as slabs and parts in which foreign matters are mixed such as discolored parts.

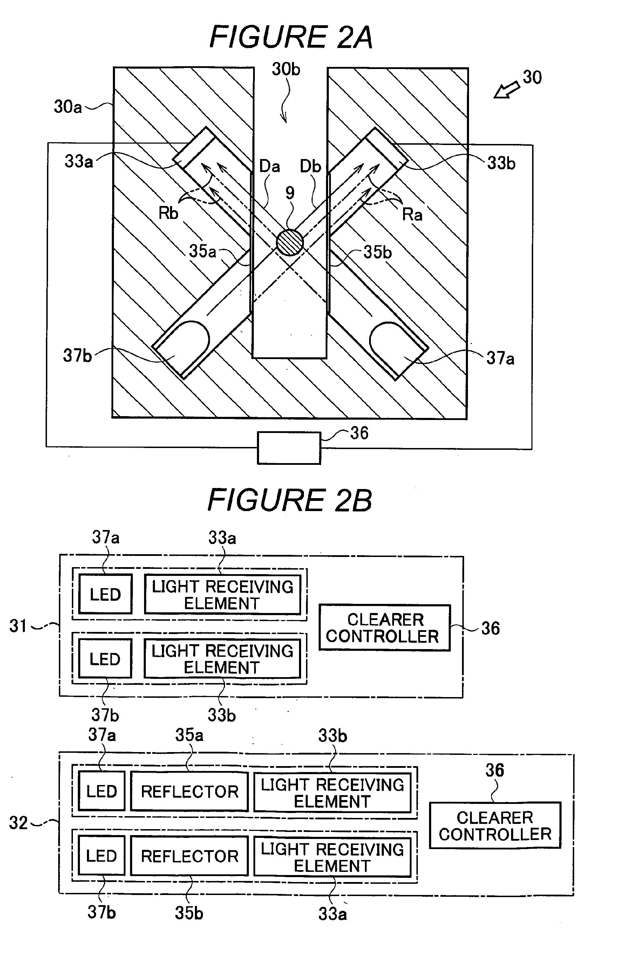

As shown in Figure 2A, an opening 30b is formed in a casing 30a of the yarn defect detecting device 30 so that the spun yarn 9 can be inserted in the opening 30b. In Figure 2A, the direction perpendicular to the sheet of the drawing corresponds to the direction in which the spun yarn 9 placed in the opening 30b moves. A pair of LEDs 37a, 37b, a pair of light receiving elements 33a, 33b, and a pair of reflectors 35a, 35b are arranged outside the spun yarn 9 in its radial direction. Across the spun yarn 9 placed in the opening 30b, the LED 37a and the light receiving element 33a lie opposite each other and the LED 37b and the light receiving element 33b lie opposite each other.

As seen in the moving direction of the spun yarn 9, in addition to the opening 30b, an X-shaped opening is formed in the casing 30a, and the LEDs 37a, 37b and light receiving elements 33a, 33b are arranged at the respective positions of ends of the X-shape. The LEDs 37a, 37b and light receiving elements 33a, 33b are each enclosed by the casing 30a except for their sides facing the spun yarn 9 placed in the opening 30b.

The yarn defect detecting device 30 comprises a clearer controller 36 to which output signals from the light receiving elements 33a, 33b are input.

As shown in Figure 2B, an abnormal yarn thickness detecting device 31 is composed of the LED 37a and light receiving element 33a, the LED 37b and light receiving element 33b, and the clearer controller 36. A foreign matter detecting device 32 is composed of the LED 37a, reflector 35a and light receiving element 33a, and LED 37b, reflector 35b and light receiving element 33b, and clearer controller 36. These devices will be described below in detail.

The abnormal yarn thickness detecting device 31 will be described.

This yarn thickness detecting device is means for detecting whether or not an abnormal thickness such as a slab is occurring in the spun yarn 9. In the present embodiment, this yarn thickness detecting device is configured as an optical detecting device.

The abnormal yarn thickness detecting device 31 has two sets each of the LED and light receiving element arranged opposite each other across the moving path of the spun yarn 9, that is, the LED 37a and light receiving element 33a, and the LED 37b and light receiving element 33b. Direct light Da radiated by the LED 37a is received by the light receiving element 33a while being partly blocked by the spun yarn 9, and direct light Db radiated by the LED 37b is received by the light receiving element 33b while being partly blocked by the spun yarn 9. Intensity information on the direct light Da, Db output by the light receiving elements 33a, 33b provides information on yarn thickness.

LEDs 37a, 37b are controlled by a time division circuit provided in the clearer controller 36 so that the LEDs 37a, 37b alternately emit light. The spun yarn 9 is irradiated with light from two different directions to ensure that yarn thickness information can be obtained even if any particular part of the spun yarn 9 is flat.

With the above configuration, the intensity of the direct light Da, Db received by the light receiving elements 33a, 33b varies depending on the yarn thickness. Then, on the basis of the intensity information on the received light intensity output by the light receiving elements 33a, 33b, the clearer controller 36 detects the presence or absence of an abnormal yarn thickness such as a slab.

The abnormal yarn thickness detecting device 31 may be a capacitance type detecting device that provides outputs corresponding to the sectional area of the yarn.

The foreign matter detecting device 32 will be described.

The foreign matter detecting device 32 is means for detecting the presence or absence of a foreign matter mixed in the spun yarn 9. Foreign matter that the foreign matter detecting device 32 is able to detect refers to those of the matters mixed in the spun yarn 9 which have colors different from that (white) of normal cotton fibers. A matter having a color different from that of normal cotton fibers has a reflectivity different from that of the normal cotton fibers. Thus, the foreign matter detecting device 32 utilizes a variation in reflected light intensity which depends on reflectivity to detect a foreign matter mixed in the spun yarn 9. The foreign matter will be described below in further detail.

The foreign matter detecting device 32 has two sets each of the LED, reflector, and light receiving element arranged opposite one another across the moving path of the spun yarn 9, that is, the LED 37a, reflector 35a and light receiving element 33b, and the LED 37b, reflector 35b, light receiving element 33a. The reflector 35a is placed opposite both LED 37a and light receiving element 33b across the moving path of the spun yarn 9. Similarly, the reflector 35b is placed opposite both LED 37b and light receiving element 33a across the moving path of the spun yarn 9.

The direct light Da radiated by the LED 37a is reflected by the spun yarn 9 and reflector 35a, and the reflected light Ra is received by the light receiving element 33b. Similarly, the direct light Db radiated by the LED 37b is reflected by the spun yarn 9 and reflector 35b, and the reflected light Rb is received by the light receiving element 33a. Reflected light intensity information output by the light receiving elements 33a, 33b provides information on the mixture of a foreign matter.

As previously described, the LEDs 37a, 37b are control led by the time division circuit provided in the clearer controller 36 so that the LEDs 37a, 37b alternately emit light. Thus, while the LED 37a is emitting light, the light emitting element 33b outputs intensity information on the reflected light Ra, and the light emitting element 33a outputs intensity information on the direct light Da. Similarly, while the LED 37b is emitting light, the light emitting element 33a outputs intensity information on the reflected light Rb, and the light emitting element 33b outputs intensity information on the direct light Db.

Also in this case, the spun yarn 9 is irradiated with light from the two different directions to ensure that information on the mixture of a foreign matter can be obtained even if a position on the spun yarn 9 at which a foreign matter is mixed is biased to the outer periphery of the spun yarn 9.

The foreign matter detecting device is configured so that the intensity of the reflected light Ra, Rb, which is incident on the light receiving elements 33a, 33b, respectively, is not affected by the yarn thickness of the spun yarn 9. Specifically, this configuration is as described below.

The LEDs 37a, 37b radiate diffusion light. The direct light Da, Db, the diffusion light, reaches the reflectors 35a, 35b, respectively, though the light is partly blocked the spun yarn 9. Thus, the intensity of the direct light Da, Db, which reaches the reflectors 35a, 35b, respectively, varies depending on the yarn thickness of the spun yarn 9. Thus, the layout of the reflectors 35a, 35b and light receiving elements 33a, 33b with respect to the LEDs 37a, 37b, and the shapes of the reflectors 35a, 35b and light receiving elements 33a, 33b, and the like are set so as to fix the intensity of the entire reflected light Ra, Rb, that is, the sum of the intensity of the reflected light Ra, Rb reflected by the reflectors 35a, 35b and the intensity of the reflected light Ra, Rb reflected by the spun yarn 9. Where no foreign matter is mixed and only the yarn thickness of the spun yarn 9 varies, the intensity of the entire reflected light Ra, Rb received by the light receiving elements 33a, 33b is prevented from varying.

Here, the surface color of the reflectors 35a, 35b is set so that the reflectivity of the spun yarn 9 for the direct light Da, Db is the same as that of the reflectors 35a, 35b for the direct light Da, Db. The surface color of the reflectors 35a, 35b is set to be white because the spun yarn 9 is formed of white cotton fibers.

In Figure 2, the reflectors 35a, 35b and light receiving elements 33a, 33b appear to be on the same plane. However, in the moving direction of the spun yarn 9, the reflectors 35a, 35b are located at different positions and the light receiving elements 33a, 33b are located at different positions. As previously described, the direct light Da from the LED 37a reaches the light receiving element 33a, and the resulting reflected light Ra reaches the light receiving element 33b. The direct light Db from the LED 37b reaches the light receiving element 33b, and the resulting reflected light Rb reaches the light receiving element 33a.

The reflectors 35a, 35b may be of a semi-transmission type so that transmitted light transmitted through the reflectors 35a, 35b reach the light receiving elements 33a, 33b, whereas the reflected light Ra, Rb reflected by the reflectors 35a, 35b reaches the light receiving elements 33a, 33b.

In any way, the direct light (transmitted light) from the LED 37a is incident on the light receiving element 33a, and the reflected light from the LED 37a is incident on the light receiving element 33b. The direct light (transmitted light) from the LED 37b is incident on the light receiving element 33b, and the reflected light from the LED 37b is incident on the light receiving element 33a.

With the above configuration, in the foreign matter detecting device 32, the intensity of the reflected light Ra, Rb received by the light receiving elements 33b, 33a, respectively, varies depending on the amount of foreign matter mixed in the spun yarn 9. Then, on the basis of the information on received light intensity output by the light emitting elements 33b, 33a, the clearer controller 36 detects the presence or absence of a foreign matter mixed in the spun yarn 9.

The method by which the foreign matter detecting device 32 eliminates the adverse effect of the yarn thickness from the intensity information on the reflected light from the yarn is not limited to the fixation of intensity of the entire reflected light using the reflectors 35a, 35b.

Information on the yarn thickness may be separately detected so that the intensity information on the reflected light from the yarn can be corrected on the basis of the yarn thickness information to provide intensity information on the reflected light from the yarn which is free from the adverse effect of the yarn thickness. Detected information on the yarn thickness can be obtained utilizing, for example, the above yarn thickness detecting device (LEDs 37a, 37b, light receiving elements 33a, 33b, clearer controller 36). This configuration eliminates the reflectors.

Plural groups of the devices shown in Figure 2A (each group includes the abnormal yarn thickness detecting device 31 and foreign matter detecting device 32 but not the clearer controller 36) may be provided along the moving direction of the spun yarn 9. Then, all the output signals from the light receiving elements 33a, 33b, provided in each set of the yarn thickness detecting device 31 and foreign matter detecting device 32 (excluding the clearer controller 36), may be input to the clearer controller 36. This configuration makes it possible to improve the accuracy of detection of abnormal yarn thickness and of foreign matters performed by the clearer controller 36.

Further, in the present embodiment, the yarn thickness detecting device 31 and foreign matter detecting device 32 each comprise two sets each of the LED and light receiving element (and also reflector). However, the yarn thickness detecting device 31 and foreign matter detecting device 32 may each comprise only one set of the LED and light receiving element (and also reflector).

Foreign matters that may be mixed in the spun yarn 9 will be described.

Foreign matters in the spun yarn 9 formed of cotton fibers refer to those of the matters mixed in the spun yarn 9 which include trash of stems or leaves of cotton fibers and discolored fibers. Trash is yellow or yellowish orange, and discolored fibers have a shade of black (black, blue, or red). The colors of these foreign matters are different from the colors (white) of normal cotton fibers. These foreign matters normally (except the case where the light source is appropriately set as described below) differ from normal cotton fibers in reflectivity. As previously described, the foreign matter detecting device 32 detects the presence or absence of a foreign matter on the basis of the difference in reflectivity and thus reflected light intensity between a part of the spun yarn 9 which contains a foreign matter and a part of the spun yarn 9 which contains no foreign matter.

Not all the foreign matters that may be mixed in normal cotton fibers need be removed in connection with the quality of the spun yarn 9. Some foreign matters may be left instead of being removed without posing any problem.

In the present embodiment, the spun yarn 9 spun by the pneumatic spinning machine 3 are dyed in a process following the spinning process. In this case, trash is dyed together with normal cotton fibers and the difference in color becomes unnoticeable. Consequently, for the pneumatic spinning machine 3, it is more economically profitable to leave trash to increase the spinning speed of the pneumatic spinning machine 3 than to determine a part mixed with trash to be a yarn defect to cut it, thus reducing the spinning speed. An arrangement described below is thus used to prevent the foreign matter detecting device 32 from recognizing trash as a foreign matter.

On the other hand, for discolored fibers having their colors of a shade of black (black, blue, or red), the changed color is noticeable even after dyeing. Consequently, these fibers need to be removed in order to improve the quality of the spun yarn 9. Thus, the foreign matter detecting device 32 is allowed to recognize discolored fibers as a foreign matter.

That is, the foreign matters are roughly classified into two types, that is, those which are desirably to be removed and those which are desirably to be left instead of being removed.

A description will be given of the arrangement that prevents the foreign matter detecting device 32 from recognizing a foreign matter to be desirably left instead of being removed, as a foreign matter.

As previously described, foreign matter detection by the foreign matter detecting device 32 is based on the difference in color (reflectivity) between normal cotton fibers and a foreign matter.

The reflectivity of a foreign matter varies in accordance with the light source color of the LEDs 37a, 37b as light sources, and thus the light receiving sensitivity of the light receiving elements 33b, 33a, which receive the reflected light Ra, Rb, varies respectively. Since normal cotton fibers are white, a variation in reflectivity caused by the light source color is negligible for the normal cotton fibers.

Where in a hue ring, the color of the light irradiation target (normal fibers or a foreign matter) has the same shade as that of the light source color, the reflectivity of the light irradiation target is close to that of a white irradiation target. On the other hand, where in the hue ring, the color of the light irradiation target (normal fibers or a foreign matter) is complementary to the light source color, the reflectivity of the light irradiation target is close to that of a black irradiation target. Where the black irradiation target is black, a reflectivity is almost zero.

Specifically, when the light source color of the LEDs 37a, 37b is green, even if a green foreign matter is mixed in the spun yarn 9, the reflected light Ra, Rb, which is incident on the light receiving elements 33b, 33a, respectively, has its intensity remaining unchanged because the reflectivity of the foreign matter is close to that of normal fibers. Consequently, the foreign matter detecting device 32 is unlikely to recognize a green foreign matter as a foreign matter.

In this case, when a red (complementary color of green) foreign matter is mixed in the spun yarn 9, the reflectivity of the foreign matter lowers closer to zero. This reduces the intensity of the reflected light Ra, Rb, which is incident on the light receiving elements 33b, 33a, respectively. The magnitude of the decrease in intensity increases consistently with the amount of red foreign matter mixed in the spun yarn 9. Consequently, the foreign matter detecting device 32 recognizes a red foreign matter as a foreign matter.

In the present embodiment, each of the LEDs 37a, 37b adopts a yellow light source.

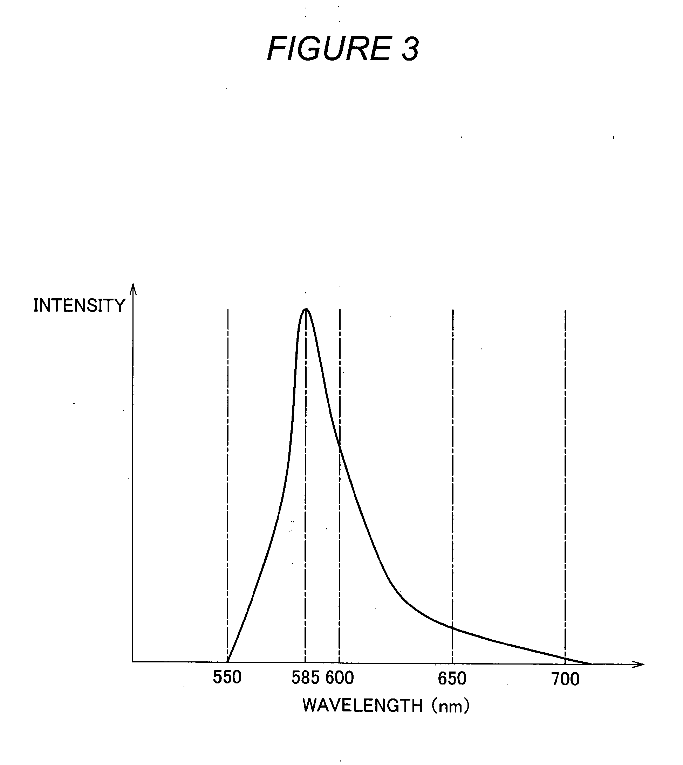

As shown in Figure 3, more specifically, radiated light from the light source has a wavelength peak value of 580 to 600 nm (nanometers). The light source also radiates light with a wavelength larger or smaller than the peak value. Accordingly, precisely speaking, the light source color of the LEDs 37a, 37b ranges from yellow to yellowish orange.

In the present embodiment, a foreign matter to be desirably left instead of being removed from the spun yarn 9 of cotton fibers is trash of stems or leaves. The color of trash of stems or leaves also ranges from yellow to yellowish orange. Thus, the light source color of the LEDs 37a, 37b is yellow (yellow to yellowish orange), and in the hue ring, is the same as or similar to the color of trash of stems or leaves. This configuration makes the foreign matter detecting device 32 unlikely to recognize trash as a foreign matter.

On the other hand, in the present embodiment, a foreign matter to be desirably removed from the spun yarn 9 of cotton fibers is a discolored fiber having its color of a shade of black (black, blue, or red). The shades of black exhibit low reflectivities regardless of the light source. Thus, the discolored fiber having its color of a shade of black (black, blue, or red) is recognized as a foreign matter by the foreign matter detecting device 32.

Then, a comparative description will be given of the case where the light source provided in the foreign matter detecting device 32 is the LEDs 37a, 37b, which are a yellow light source, and the case where a green light source is used.

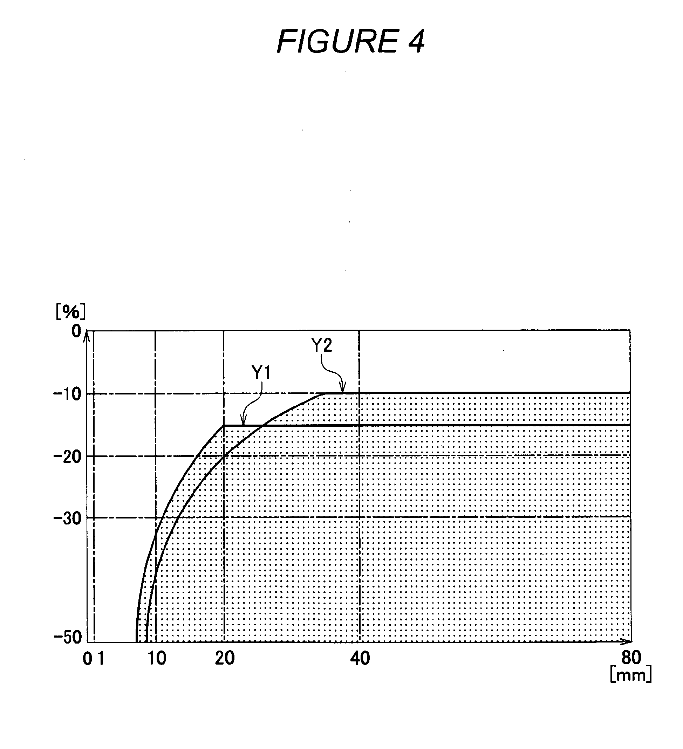

Figure 4 shows an example of how to set a threshold when the yellow light source (LEDs 37a, 37b) is used.

The axis of ordinate in Figure 4 indicates the intensity of the reflected light Ra, Rb from a part of the spun yarn 9 passing through the foreign matter detecting device 32. The intensity of the reflected light Ra, Rb obtained when there is no foreign matter (discolored fibers having their colors of a shade of black (black, blue, or red)) to be removed is set to be a reference. Where there are any foreign matters to be removed, the rate of a decrease in intensity is expressed in percentage. Further, the axis of abscissa in Figure 4 indicates the yarn length over which the same intensity (intensity of the reflected light Ra, Rb) continues. For example, where the spun yarn 9 is mixed with one discolored fiber having its color of a shade of black (black, blue, or red), a part with the same intensity (for example, -10%) is detected over a given length (for example, 30 mm) by the foreign matter detecting device 32. Here, the yarn length of the part with the same length is calculated by multiplying the detection time for which the part with the same intensity is continuously detected by the foreign matter detecting device 32, by the moving speed of the spun yarn 9.

The threshold provides a determination criterion used to determine that a part mixed with a foreign matter such as a discolored part to be a yarn defect.

Settings for this threshold include a setting Y1 (lower limit intensity: - 15%, lower limit length: 20 mm) and a setting Y2 (lower limit intensity: - 10%, lower limit length: 35 mm). The setting Y1 is a criterion on the basis of which the foreign matter detecting device 32 detects and determines a part of the spun yarn 9 exhibiting an intensity of less than -15% over at least 20 mm to be a yarn defect. The setting Y2 is a criterion on the basis of which the foreign matter detecting device 32 detects and determines a part of the spun yarn 9 exhibiting an intensity of less than -10% over at least 35 mm to be a yarn defect.

In an area shown with a dot pattern in Figure 4, the presence of a yarn defect is determined on the basis of at least one of the settings Y1, Y2.

Further, a determination criterion is set such that the intensity and the length exhibit a curved relationship at a length of 20 mm or less for the setting Y1 and at a length of 35 mm or less for the setting Y2. In this area, the presence of a yarn defect is determined where the intensity is high in spite of a short length.

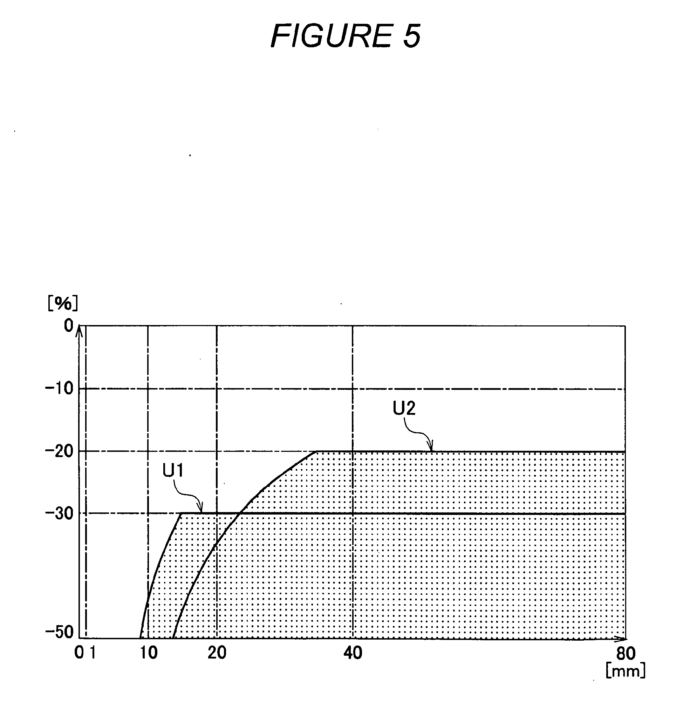

Figure 5 shows an-example of how to set a threshold when a green light source is used.

The settings for the axes of ordinate and abscissa are similar to those in the case of the yellow light source shown in Figure 4.

Further, settings for this threshold include a setting U1 (lower limit intensity: - 30%, lower limit length: 15 mm) and a setting U2 (lower limit intensity: - 20%, lower limit length: 35 mm). The threshold is set such that at a length of 15 mm or less for the setting U1 and at a length of 35 mm or less for the setting U2, the presence of a yarn defect is determined where the intensity is high in spite of a short length, as is the case with the settings Y1, Y2.

As the determination criterion for yarn defects, the threshold settings for the green light source shown in Figure 5 are looser than these for the yellow light source shown in Figure 4.

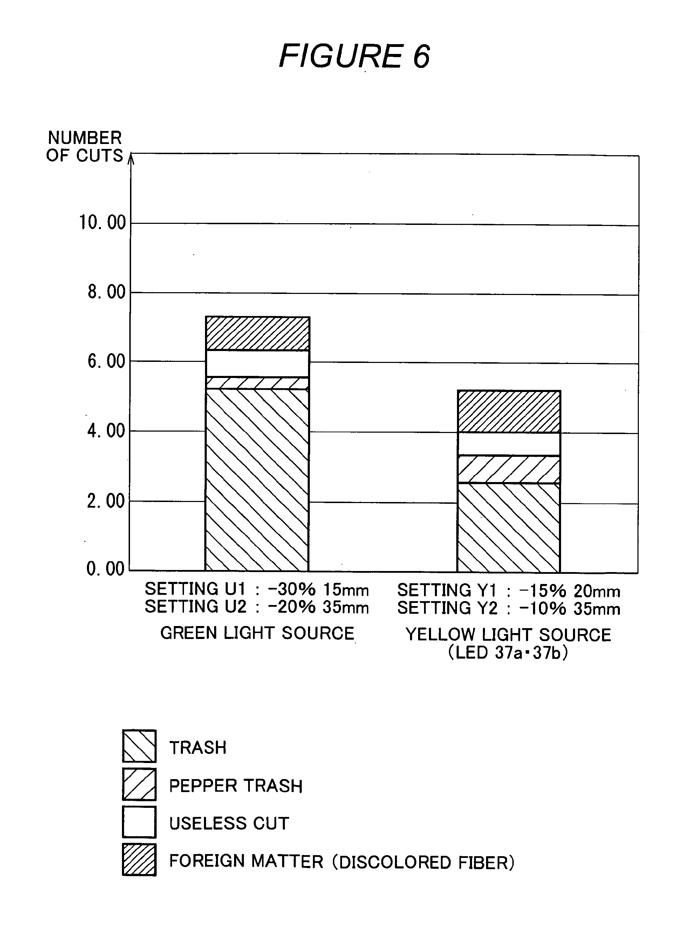

Figure 6 shows a comparison of the yellow light source with the green light source for the number of cuts.

In this case, for the spun yarn 9 manufactured from the same raw material by the pneumatic spinning machine 3, the number of cuts made within a given length (for example, 100 km) was compared between the case with the yellow light source and the case with the green light source. Accordingly, the composition of the spun yarn 9 in the case with the yellow light source can be considered to be equivalent to that in the case with the green light source.

The figure shows only the number of cuts made on yarn defects corresponding to parts mixed with foreign matters such as discolored parts and does not include the number of cuts made on yarn defects resulting from abnormal yarn thickness.

As shown in Figure 6, the number of cuts made on trash, a foreign matter to be desirably left instead of being removed, in the case with the yellow light source is about half that in the case with the green light source. Furthermore, as previously described, the case with the yellow light source used the stricter determination criterion for yarn defects than the case with the green light source.

On the other hand, for discolored fibers having their colors of a shade of black (black, blue, or red), a foreign matter to be removed, there is no difference between the case with the yellow light source and the case with the green light source.

Thus, in the hue ring, the light source color is the same as or similar to the color of the foreign matter to be desirably left instead of being removed. This makes it possible to make the foreign matter detecting device 32 unlikely to detect the foreign matter to be desirably left instead of being removed, as a foreign matter, compared to the use of a light source with another color light or light other than visible light. This in turn prevents the spinning speed of the pneumatic spinning machine 3 from decreasing and enables the proper removal of the foreign matter to be removed without degrading the quality of the spun yarn 9.

While the present invention has been described with respect to preferred embodiments thereof, it will be apparent to those skilled in the art that the disclosed invention may be modified in numerous ways and may assume many embodiments other than those specifically set out and described above. Accordingly, it is intented by the appended claims to cover all modifications of the present invention that fall within the true spirit and scope of the invention.

## Claims

1. A foreign matter detecting device which irradiates a yarn formed of white fibers with light from a light source and which measures intensity of reflected light from the yarn to detect presence or absence of a foreign matter mixed in the yarn on the basis of the intensity information, the device being **characterized in that**:
in a hue ring, a light source color of the light source is the same as or similar to a color of a foreign matter to be desirably left instead being removed.

2. A foreign matter detecting device according to Claim 1, **characterized in that** the yarn is a spun yarn formed of cotton fibers,
the foreign matter to be desirably left instead of being removed is trash of cotton leaves or stems which is yellow or yellowish orange, and
the light source color is yellow or yellowish orange.

3. A textile machine **characterized by** comprising:
a foreign matter detecting device according to Claim 1 or Claim 2;
a cutting device for the yarn which operates on the basis of detection of the foreign matter by the foreign matter detecting device; and
a winding device that winds the yarn.

4. A foreign matter detecting method of irradiating a yarn formed of white fibers with light from a light source and measuring intensity of reflected light from the yarn to detect presence or absence of a foreign matter mixed in the yarn on the basis of the intensity information, the method being **characterized in that**:
in a hue ring, a light source color of the light source is the same as or similar to a color of foreign matter to be desirably left instead being removed.
